# EUROPÄISCHE PATENTSCHRIFT

(11) **EP 4 093 724 B1**
(45) Veröffentlichungstag und Bekanntmachung des Hinweises auf die Patenterteilung: **31.01.2024**
(21) Anmeldenummer: 20811684.8
(22) Anmeldetag: 30.11.2020
(51) Int. Cl.: C07C 29/70, C07C 31/30

(54) **VERFAHREN ZUR GLEICHZEITIGEN HERSTELLUNG VON NATRIUM- UND KALIUMALKOHOLATEN**
METHOD FOR THE SIMULTANEOUS PREPARATION OF SODIUM AND POTASSIUM ALCOHOLATES
PROCÉDÉ DE FABRICATION SIMULTANÉE D'ALCOOLS DE SODIUM ET DE POTASSIUM

(30) Priorität: 23.01.2020 EP 20153334
(43) Veröffentlichungstag der Anmeldung: 30.11.2022
(73) Patentinhaber: Evonik Operations GmbH, 45128 Essen (DE)
(72) Erfinder: ROETTGER, Dirk, 50672 Köln (DE); REIMANN, Sebastian, 50389 Wesseling (DE); NEUMANN, Manfred, 45770 Marl (DE); SCHRÖDER, Moritz, 48153 Münster (DE); ZITZEWITZ, Philip, 45721 Haltern am See (DE); PAUL, Niklas, 45770 Marl (DE); RIX, Armin Matthias, 45770 Marl (DE)
(74) Vertreter: Evonik Patent Association
(86) Internationale Anmeldenummer: PCT/EP2020/083877
(87) Internationale Veröffentlichungsnummer: WO 2021/148174

(56) Entgegenhaltungen:
- CN-U- 208 632 416
- DE-C- 968 903

## Beschreibung

Die vorliegende Erfindung betrifft ein Verfahren zur Herstellung von Natrium- und Kaliumalkoholaten. Es zeichnet sich durch zwei gleichzeitig, aber räumlich getrennt durchgeführte Umsetzungen eines Alkohols ROH mit NaOH und KOH zu Natrium- bzw. Kaliumalkoholat aus. Die dabei entstehenden Brüden umfassen den eingesetzten Alkohol und Wasser. Sie werden vereint und der erhaltene Mischbrüden einer gemeinsamen Destillation unter Rückgewinnung des Alkohols zugeführt.

### Hintergrund der Erfindung

Alkalimetallalkoholate werden als starke Basen in der Synthese zahlreicher Chemikalien, z.B. bei der Herstellung von Pharma- oder Agrowirkstoffen, eingesetzt. Weiterhin finden Alkalimetallalkoholate Anwendung als Katalysatoren in Umesterungs- und Amidierungsreaktionen.

Alkalimetallalkoholate (MOR) werden mittels Reaktivdestillation in einer Gegenstromdestillationskolonne aus Alkalimetallhydroxiden (MOH) und Alkoholen (ROH) hergestellt, wobei das gemäß folgender Reaktion entstehende Reaktionswasser mit dem Destillat entfernt wird.

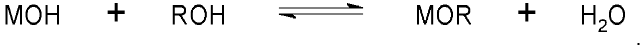

Ein solches Verfahrensprinzip ist beispielsweise in der US 2,877,274 A beschrieben, wobei wässrige Alkalimetallhydroxid-Lösung und gasförmiges Methanol im Gegenstrom in einer Rektifikationskolonne gefahren werden. In prinzipiell nicht veränderter Form wird dieses Verfahren in der WO 01/42178 A1 erneut beschrieben.

Ähnliche Verfahren, bei denen jedoch zusätzlich noch ein Schleppmittel, wie beispielsweise Benzol, eingesetzt wird, sind in der GB 377,631 A und der US 1,910,331 A beschrieben. Das Schleppmittel dient hierbei zur Trennung von Wasser und dem wasserlöslichen Alkohol. Bei beiden Patentschriften wird das Kondensat einer Phasentrennung unterzogen, um das Reaktionswasser abzutrennen.

Entsprechend beschreibt die DE 96 89 03 C ein Verfahren zur kontinuierlichen Herstellung von Alkalialkoholaten in einer Reaktionskolonne, wobei das am Kopf der Kolonne entnommene Wasser-Alkohol-Gemisch kondensiert und anschließend einer Phasentrennung unterworfen wird. Die wässrige Phase wird hierbei verworfen und die alkoholische Phase zusammen mit dem frischen Alkohol der Kolonne am Kopf zurückgegeben. Ein ähnliches Verfahren beschreibt die EP 0 299 577 A2, wobei die Wasserabtrennung im Kondensat mit Hilfe einer Membran erfolgt.

Die industriell wichtigsten Alkalimetallalkoholate sind jene des Natriums und Kaliums, und hierbei insbesondere die Methylate und Ethylate. Deren Synthese ist vielfach im Stand der Technik beschrieben, zum Beispiel in der EP 1 997 794 A1.

Auf einigen technischen Gebieten, zum Beispiel bei der Herstellung von Biodiesel, besteht ein Bedarf nach beiden Alkoholaten, jenen des Kaliums sowie auch jenen des Natriums (vergleiche z.B. A.B.M.S. Hossain, M.A. Mazen, Australian Journal of Crop Science 2010, 4 (7), 550-555).

Es besteht deshalb ein Bedürfnis nach Verfahren zur effizienten Herstellung von Alkoholaten beider Alkalimetalle.

Im Stand der Technik wurden Verfahren beschrieben, mit denen sich im Prinzip Alkoholate beider Alkalimetalle, des Natriums und des Kaliums, herstellen lassen.

So beschreibt CN 208632416 U ein Verfahren zur kontinuierlichen Herstellung von Lösungen des Natriummethanolats (im Folgenden "NM") und Kaliummethanolats (im Folgenden "KM") via Reaktivrektifikation. Dieses Verfahren zeichnet sich dadurch aus, dass schneller zwischen der Umsetzung zu NM bzw. KM gewechselt werden kann. Allerdings kann in einer bestimmten Zeit nur entweder NM oder KM kontinuierlich hergestellt werden. Dies ist an sich schon wirtschaftlich nachteilig. Daneben kommt es in einer solchen Multi-purpose Anlage beim Produktwechsel zu Produktverunreinigung und/oder-verlust, so dass NM-Verunreinigungen in KM auftreten und umgekehrt. Um diese zu minimieren, ist bei jedem Wechsel des Alkalimetalls eine aufwendige Reinigung der Anlage nötig.

Die Aufgabe der vorliegenden Erfindung bestand demnach darin, ein verbessertes Verfahren zur Verfügung zu stellen, welches eine effiziente Herstellung von Natrium- und Kaliumalkoholaten erlaubt. Ein solches Verfahren soll insbesondere die Nachteile der im Stand der Technik beschriebenen Verfahren, wie die Querkontamination des einen Alkalimetallalkoholats mit dem anderen und den Produktverlust beim Umstellen des Eduktes, beheben.

Es wurde nun überraschenderweise ein Verfahren gefunden, dass die erfindungsgemäße Aufgabe löst.

### Kurzzusammenfassung der Erfindung

Das erfindungsgemäße, bevorzugt kontinuierliche, Verfahren ist eines zur Herstellung von Natriumalkoholat der Formel RONa und Kaliumalkoholat der Formel ROK, wobei
(a) ROH mit NaOH im Gegenstrom umgesetzt wird, wodurch RONa und ein Brüden **B₁** umfassend Wasser und ROH erhalten werden,
(b) ROH mit KOH im Gegenstrom umgesetzt wird, wodurch ROK und ein Brüden **B₂** umfassend Wasser und ROH erhalten werden,

wobei Rein C₁-C₆-Kohlenwasserstoffrest ist,
dadurch gekennzeichnet,
dass die Umsetzungen nach (a) und (b) gleichzeitig und räumlich getrennt ablaufen,
und dass die Brüden **B₁** und **B₂** mindestens teilweise zum gemischten Brüden **B_{M}** umfassend ROH und Wasser gemischt werden,
und dass ROH mindestens teilweise durch Destillation aus **B_{M}** abgetrennt wird.

### Abbildungen

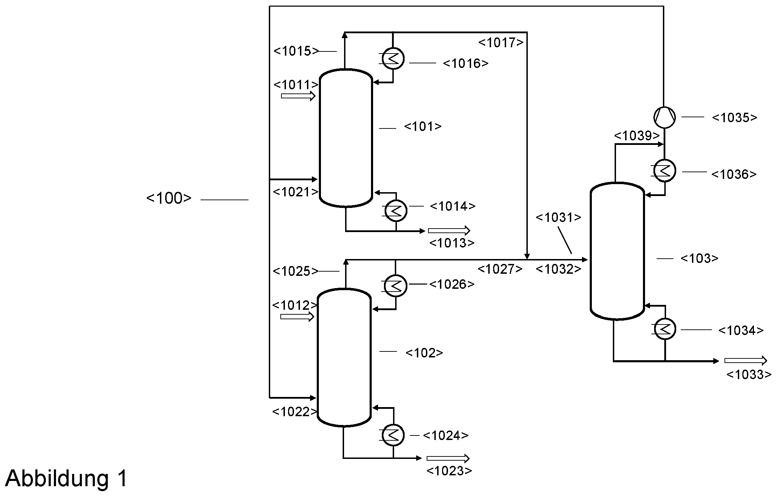

Abbildung 1 zeigt eine Ausführungsform des erfindungsgemäßen Verfahrens, in welcher NaOH <1011 > und KOH <1012> jeweils in zwei getrennten Reaktionskolonnen <101 >, <102> mit Methanol <1021 > bzw. <1022> zum jeweiligen Methanolat <1013> bzw. <1023> umgesetzt werden. Die erhaltenen Brüden <1015> und <1025> werden als Mischung <1031> einer Wasser/Methanol-Kolonne <103> zugeführt, in welcher Methanol <1039> destillativ rückgewonnen wird.

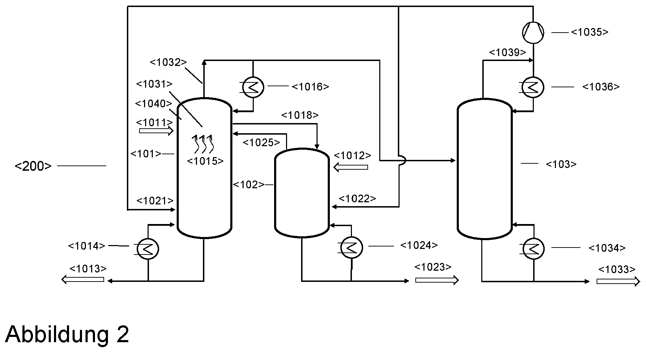

Abbildung 2 zeigt eine Ausführungsform des erfindungsgemäßen Verfahrens, in welcher NaOH <1011 > und KOH <1012> jeweils in zwei getrennten Reaktionskolonnen <101 >, <102> mit Methanol <1021 > bzw. <1022> zum jeweiligen Methanolat <1013> bzw. <1023> umgesetzt werden. Die KOH-Kolonne <102> ist dabei als Seitenkolonne der NaOH-Kolonne <101> ausgestaltet. Die erhaltenen Brüden <1015> und <1025> werden im oberen Bereich der Kolonne <101 > gemischt. Die am Kopf der Kolonne <101 > erhaltene Mischung <1031 > wird einer Wasser/Methanol-Kolonne <103> zugeführt, in welcher Methanol <1039> destillativ rückgewonnen wird.

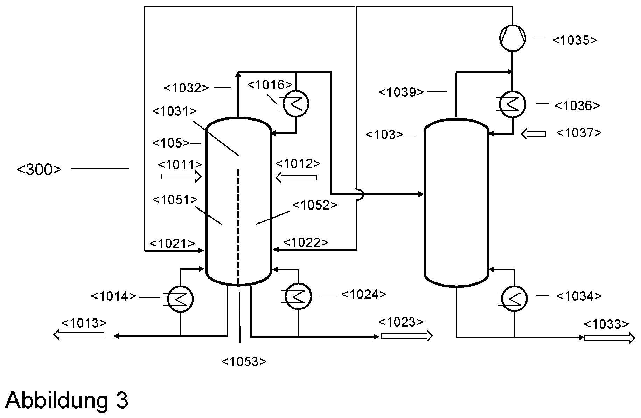

Abbildung 3 zeigt eine Ausführungsform des erfindungsgemäßen Verfahrens, in welcher NaOH <1011 > und KOH <1012> mit Methanol <1021 > bzw. <1022> in einer einzigen Reaktionskolonne <105> mit nach unten durchgezogener Trennwand <1053> zum jeweiligen Methanolat <1013> bzw. <1023> umgesetzt werden. Die Zugabe von NaOH <1011 > und KOH <1012> zur Reaktionskolonne <105> erfolgt unterhalb des oberen Randes auf jeweils unterschiedlichen Seiten der Trennwand <1053>. Die erhaltenen Brüden werden als Mischung <1031 > einer Wasser/Methanol-Kolonne <103> zugeführt, in welcher Methanol <1039> destillativ rückgewonnen wird.

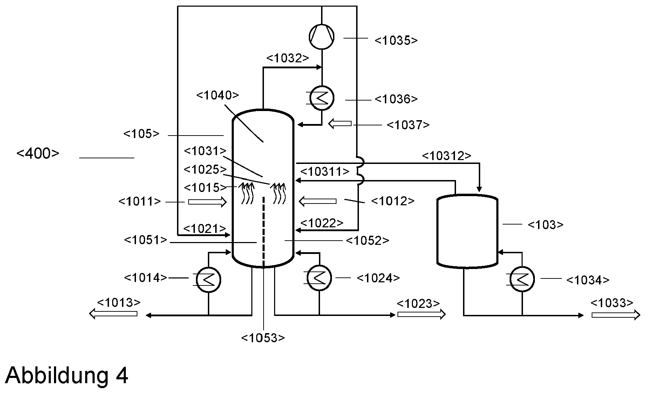

Abbildung 4 zeigt eine Ausführungsform des erfindungsgemäßen Verfahrens, in welchem NaOH <1011 > und KOH <1012> mit Methanol <1021 > bzw. <1022> in einer einzigen Reaktionskolonne <105> mit nach unten durchgezogener Trennwand <1053> und Verstärkungsteil <1040> zum jeweiligen Methanolat <1013> bzw. <1023> umgesetzt werden. Die destillative Rückgewinnung von Methanol aus den Brüden <1015>, <1025> aus den Reaktionsräumen <1051> bzw. <1052> auf beiden Seiten der Trennwand <1053> erfolgt im Verstärkungsteil <1040> der Reaktionskolonne <105> und dem als Seitenkolonne <103> ausgeführten Abtriebsteil.

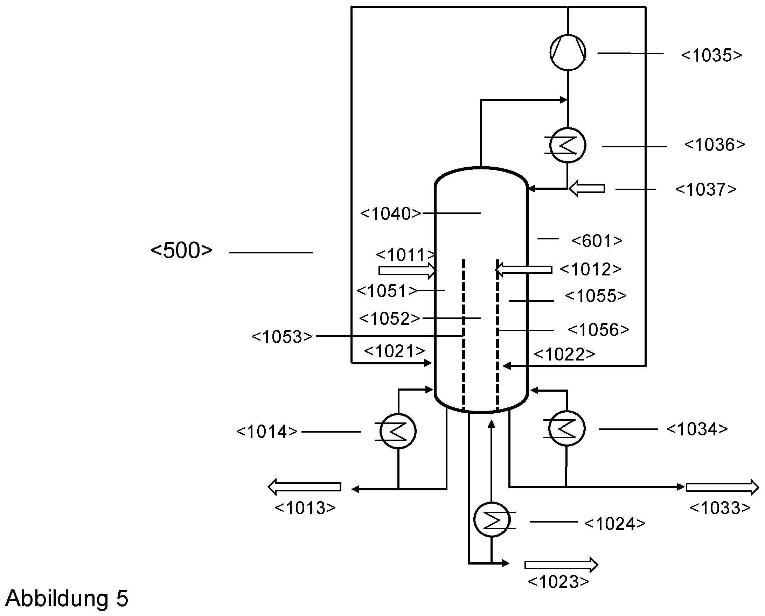

Abbildung 5 zeigt eine Ausführungsform des erfindungsgemäßen Verfahrens, in welchem NaOH <1011 > und KOH <1012> mit Methanol <1021 > bzw. <1022> in einer einzigen Reaktionskolonne <601> mit zwei nach unten durchgezogenen Trennwänden <1053>, <1056> und Verstärkungsteil <1040> zum jeweiligen Methanolat <1013> bzw. <1023> umgesetzt werden. Die erhaltenen Brüden werden im Verstärkungsteil <1040> und im Abtriebsteil <1055> der Reaktionskolonne <105> durch Destillation aufgetrennt.

### Detaillierte Beschreibung der Erfindung

Das erfindungsgemäße Verfahren zeichnet sich dadurch aus, dass das Natriumalkoholat und das Kaliumalkoholat gleichzeitig und räumlich getrennt hergestellt werden und die jeweils anfallenden Brüden, welche Wasser und den Alkohol ROH umfassen, als Mischung einer Destillation zur Abtrennung und insbesondere Rückgewinnung des Alkohols zugeführt werden. Dies erlaubt eine besonders effiziente Herstellung der jeweiligen Alkoholate, da eine Reinigung der jeweiligen Reaktionskolonne nicht nötig ist und die Verunreinigungen von beiden Alkoholaten RONa in ROK dadurch vermieden werden. Daneben bietet das erfindungsgemäße Verfahren den Vorteil, dass nicht wie bei den Verfahren des Standes der Technik zwischen den Kampagnen hin- und hergewechselt werden muss, sondern gleichzeitig beide Alkoholate RONa und ROK hergestellt werden können.

In Schritt (a) und Schritt (b) des erfindungsgemäßen Verfahrens wird jeweils der Alkohol ROH mit NaOH bzw. KOH im Gegenstrom umgesetzt, wodurch das entsprechende Natriumalkoholat der Formel RONa und ein Brüden **B₁** bzw. das entsprechende Kaliumalkoholat der Formel ROK und ein Brüden **B₂** erhalten werden. Die Brüden **B₁** und **B₂** umfassen jeweils ROH und Wasser. Diese werden mindestens teilweise zum gemischten Brüden **B_{M}** umfassend ROH und Wasser gemischt. Aus diesem wird ROH durch Destillation mindestens teilweise abgetrennt.

In einer bevorzugten Ausführungsform der vorliegenden Erfindung wird der so aus **B_{M}** abgetrennte Alkohol ROH wieder in Umsetzung (a) und/oder Umsetzung (b), bevorzugt in beiden Umsetzungen (a) und (b), eingesetzt.

R ist im erfindungsgemäßen Verfahren ein C₁-C₆-Kohlenwasserstoffrest, bevorzugt ausgewählt aus der Guppe bestehend aus Methyl, Ethyl, *n*-Propyl, *iso*-Propyl, *n*-Butyl, *sec*-Butyl, *iso*-Butyl, tert-Butyl, Isomeren des Pentyls, wie beispielsweise *n*-Pentyl, bevorzugter ausgewählt aus der Gruppe bestehend aus Methyl, Ethyl, *n*-Propyl, *iso*-Propyl, noch bevorzugter ausgewählt aus der Gruppe bestehend aus Methyl, Ethyl. Besonders bevorzugt ist R Methyl und ROH demnach Methanol.

In einer bevorzugten Ausführungsform des erfindungsgemäßen Verfahrens wird NaOH in der Umsetzung in Schritt (a) und/oder KOH in der Umsetzung nach (b) in mindestens einem Lösungsmittel ausgewählt aus Wasser, ROH, bevorzugt in einer Mischung aus ROH und Wasser gelöst, zugegeben.

Das bedeutet, dass bevorzugt in Schritt (a) des erfindungsgemäßen Verfahrens NaOH in Wasser, in ROH oder in einer Wasser/ROH-Mischung zugegeben wird, wobei die Zugabe in Wasser oder ROH bevorzugter und die Zugabe in Wasser besonders bevorzugt ist.

Das bedeutet, dass bevorzugt in Schritt (b) des erfindungsgemäßen Verfahrens KOH in Wasser, in ROH oder in einer Wasser/ROH-Mischung zugegeben wird, wobei die Zugabe in Wasser oder ROH bevorzugter und die Zugabe in Wasser besonders bevorzugt ist.

Wird in Schritt (a) des erfindungsgemäßen Verfahrens NaOH in Wasser zugegeben, liegt der Massenanteil von NaOH, bezogen auf das Gesamtgewicht der wässrigen Lösung, insbesondere im Bereich von 10 bis 55 Gewichts-%, bevorzugt 15 bis 54 Gewichts-%, bevorzugter von 30 bis 53 Gewichts-% und besonders bevorzugt von 45 bis 52 Gewichts-%. Am bevorzugtesten liegt der Anteil von NaOH in der wässrigen Lösung dann bei 50 Gewichts-%.

Wird in Schritt (b) des erfindungsgemäßen Verfahrens KOH in Wasser zugegeben, liegt der Massenanteil von KOH, bezogen auf das Gesamtgewicht der wässrigen Lösung, insbesondere im Bereich von 10 bis 55 Gewichts-%, bevorzugt 15 bis 54 Gewichts-%, bevorzugter von 30 bis 53 Gewichts-% und besonders bevorzugt von 45 bis 52 Gewichts-%. Am bevorzugtesten liegt der Anteil von KOH in der wässrigen Lösung dann bei 50 Gewichts-%.

Der in dem erfindungsgemäßen Verfahren eingesetzte Alkohol ROH kann sowohl als Lösemittel als auch als Edukt dienen. Weiterhin kann der eingesetzte Alkohol ROH dazu dienen, Wasser aus dem jeweiligen Umsetzungsgemisch herauszustrippen, so dass dieses über das Destillat entfernt werden kann.

In einer bevorzugten Ausführungsform des erfindungsgemäßen Verfahrens, und insbesondere in den Fällen, in denen NaOH in Schritt (a) in Wasser zugegeben wird, beträgt das Verhältnis des Gesamtgewichts (Massen; Einheit: kg) an in Schritt (a) eingesetztem Alkohol ROH zum Gesamtgewicht (Massen; Einheit: kg) an in Schritt (a) eingesetzten NaOH 4 : 1 bis 50 : 1, bevorzugter 9 : 1 bis 48 : 1, noch bevorzugter 13 : 1 bis 35 : 1.

In dieser bevorzugten Ausführungsform des erfindungsgemäßen Verfahrens, und insbesondere in den Fällen, in denen KOH in Schritt (b) in Wasser zugegeben werden, beträgt das Verhältnis des Gesamtgewichts (Massen; Einheit: kg) an in Schritt (b) eingesetztem Alkohol ROH zum Gesamtgewicht (Massen; Einheit: kg) an in Schritt (b) eingesetzten KOH 4 : 1 bis 50 : 1, bevorzugter 9 : 1 bis 48 : 1, noch bevorzugter 13 : 1 bis 35 : 1.

Werden NaOH oder KOH in Schritt (a) bzw. (b) des erfindungsgemäßen Verfahrens in ROH oder in einer Mischung aus ROH und Wasser zugegeben, handelt es sich bei dem Alkohol ROH bevorzugt um denjenigen, mit dem NaOH und KOH in der Umsetzung (a) bzw. (b) umgesetzt werden soll.

Wird NaOH in Schritt (a) des erfindungsgemäßen Verfahrens in ROH zugegeben, liegt der Massenanteil von NaOH in ROH, bezogen auf das Gesamtgewicht der Lösung, insbesondere im Bereich von 10 bis 55 Gewichts-%, bevorzugt 15 bis 54 Gewichts-%, bevorzugter von 30 bis 53 Gewichts-%, und besonders bevorzugt von 45 bis 52 Gewichts-%.

Wird KOH in Schritt (b) des erfindungsgemäßen Verfahrens in ROH zugegeben, liegt der Massenanteil von KOH in ROH, bezogen auf das Gesamtgewicht der Lösung, insbesondere im Bereich von 10 bis 55 Gewichts-%, bevorzugt 15 bis 54 Gewichts-%, bevorzugter von 30 bis 53 Gewichts-%, und besonders bevorzugt von 45 bis 52 Gewichts-%.

Wird NaOH in Schritt (a) des erfindungsgemäßen Verfahrens in einer Mischung aus Wasser und ROH zugegeben, liegt der Massenanteil von NaOH in ROH und Wasser, bezogen auf das Gesamtgewicht der Lösung, insbesondere im Bereich von 10 bis 55 Gewichts-%, bevorzugt 15 bis 54 Gewichts-%, bevorzugter von 30 bis 53 Gewichts-%, und besonders bevorzugt von 45 bis 52 Gewichts-%.

Wird KOH in Schritt (b) des erfindungsgemäßen Verfahrens in einer Mischung aus Wasser und ROH zugegeben, liegt der Massenanteil von KOH in ROH und Wasser, bezogen auf das Gesamtgewicht der Lösung, insbesondere im Bereich von 10 bis 55 Gewichts-%, bevorzugt 15 bis 54 Gewichts-%, bevorzugter von 30 bis 53 Gewichts-%, und besonders bevorzugt von 45 bis 52 Gewichts-%.

Der in Schritt (a) und in Schritt (b) des erfindungsgemäßen Verfahrens eingesetzte Alkohol ROH kann bei der erfindungsgemäßen Ausgestaltung des Verfahrens auch handelsüblicher Alkohol mit einem Alkoholmassenanteil von mehr als 99.8 Gewichts-% und einem Massenanteil an Wasser von bis zu 0.2 Gewichts-% sein.

Die Umsetzungen in Schritt (a) und Schritt (b) des erfindungsgemäßen Verfahrens werden insbesondere bei einer Temperatur im Bereich von 45 °C bis 150 °C, bevorzugt 47 °C bis 120 °C, bevorzugter 60 °C bis 110°C, und bei einem Druck von 0.5 bar bis 40 bar, bevorzugt im Bereich von 0.75 bar bis 5 bar, bevorzugter im Bereich von 1 bar bis 2 bar, bevorzugter im Bereich von 1 bar bis 1.5 bar, noch bevorzugter bei Umgebungsdruck (1 bar), durchgeführt.

Die Schritte (a) und (b) des erfindungsgemäßen Verfahrens werden räumlich getrennt durchgeführt, wobei die Brüden **B₁** und **B₂** mindestens teilweise zum gemischten Brüden **B_{M}** umfassend ROH und Wasser gemischt werden und ROH mindestens teilweise durch Destillation aus **B_{M}** abgetrennt wird.

Die mindestens teilweise Mischung der Brüden **B₁** und **B₂** zum gemischten Brüden **B_{M}** umfassend ROH und Wasser ist nicht besonders beschränkt und gemäß dem Fachmann bekannten Verfahren möglich. Es genügt insbesondere, die Brüden **B₁** und **B₂** mindestens teilweise zum gemischten Brüden **B_{M}** zusammenzuführen. Aus diesem wird dann nach dem Fachmann bekannten Verfahren, zum Beispiel in einer Rektifikationskolonne, ROH abgetrennt.

Der aus **B_{M}** abgetrennte Alkohol ROH wird insbesondere wieder in Umsetzung (a) und/oder Umsetzung (b), bevorzugt in Umsetzung (a) und Umsetzung (b), eingesetzt.

Entscheidend ist, dass das erfindungsgemäße Verfahren räumlich getrennt und gleichzeitig durchgeführt wird. Im Folgenden sind mehrere Ausführungsformen beschrieben, gemäß welchen diese räumliche Trennung bei gleichzeitiger Umsetzung bevorzugt erreicht wird.

In einer ersten bevorzugten Ausführungsform wird diese räumlich getrennte, gleichzeitige Umsetzung dadurch gewährleistet, dass die Umsetzung nach (a) in einer ersten Reaktionskolonne und die Umsetzung nach (b) in einer von der ersten verschiedenen, zweiten Reaktionskolonne durchgeführt werden.

In einer zweiten bevorzugten Ausführungsform wird diese räumlich getrennte, gleichzeitige Umsetzung dadurch gewährleistet, dass die Umsetzung nach (a) und die Umsetzung nach (b) in derselben Reaktionskolonne, in welcher sie durch mindestens eine Trennwand voneinander getrennt sind, durchgeführt werden.

In einer dritten bevorzugten Ausführungsform wird diese räumlich getrennte, gleichzeitige Umsetzung dadurch gewährleistet, dass das erfindungsgemäße Verfahren in einer Reaktionskolonne, welche eine Trennwand und bevorzugt einen Verstärkungsteil umfasst, und einer Seitenkolonne der Reaktionskolonne durchgeführt wird, wobei einer der beiden Schritte (a) und (b) in einem der durch die Trennwand abgegrenzten Bereiche der Reaktionskolonne durchgeführt wird, und der andere der beiden Schritte (a) und (b) in der Seitenkolonne durchgeführt wird, und die mindestens teilweise destillative Abtrennung von ROH aus **B_{M}** in dem durch die Trennwand abgegrenzten Bereich der Reaktionskolonne, in dem keiner der Schritte (a), (b) durchgeführt wird, und bevorzugt dem Verstärkungsteil, erfolgt.

Bevorzugt wird die in Schritt (a) erhaltene RONa als Lösung **L_{NM}** von RONa in ROH erhalten. Noch bevorzugter weist **L_{NM}** dann einen Massenanteil von RONa in ROH im Bereich 1 bis 50 Gew.-%, bevorzugt 5 bis 32 Gew.-%, bevorzugter 15 bis 32 Gew.-%, am bevorzugtesten 30 bis 32 Gew.-% auf, jeweils bezogen auf die gesamte Lösung **L_{Nm}.** Der Massenanteil von RONa in ROH kann dabei mit dem Fachmann geläufigen Mitteln, zum Beispiel einem zusätzlichen Verdampfer am Sumpf einer Reaktionskolonne, eingestellt werden. Der Massenanteil an Restwasser in **L_{NM}** liegt dabei bevorzugt bei < 1 Gew.-%, bevorzugt < 0.1 Gew.-%, bevorzugter < 0.01 Gew.-%, jeweils bezogen auf die gesamte Lösung **L_{NM}.** Der Massenanteil an NaOH in **L_{NM}** liegt dabei bevorzugt bei < 1 Gew.-%, bevorzugt < 0.1 Gew.-%, bevorzugter < 0.01 Gew.-%, jeweils bezogen auf die gesamte Lösung **L_{NM}.**

Bevorzugt wird die in Schritt (b) erhaltene ROK als Lösung **L_{KM}** von ROK in ROH erhalten. Noch bevorzugter weist **L_{KM}** dann einen Massenanteil von ROK in ROH im Bereich 1 bis 50 Gew.-%, bevorzugt 5 bis 32 Gew.-%, bevorzugter 15 bis 32 Gew.-%, am bevorzugtesten 30 bis 32 Gew.-% auf, jeweils bezogen auf die gesamte Lösung **L_{KM}.** Der Massenanteil von ROK in ROH kann dabei mit dem Fachmann geläufigen Mitteln, zum Beispiel einem zusätzlichen Verdampfer am Sumpf einer Reaktionskolonne, eingestellt werden. Der Massenanteil an Restwasser in **L_{KM}** liegt dabei bevorzugt bei < 1 Gew.-%, bevorzugt < 0.1 Gew.-%, bevorzugter < 0.01 Gew.-%, jeweils bezogen auf die gesamte Lösung **L_{KM}.** Der Massenanteil an KOH in **L_{KM}** liegt dabei bevorzugt bei < 1 Gew.-%, bevorzugt < 0.1 Gew.-%, bevorzugter < 0.01 Gew.-%, jeweils bezogen auf die gesamte Lösung **L_{KM}.**

### 1. Erste bevorzugte Ausführungsform: (a) und (b) in jeweils verschiedenen

### Reaktionskolonnen

Die räumliche Trennung der Umsetzungen (a) und (b) wird in einer ersten bevorzugten Ausführungsform des erfindungsgemäßen Verfahrens dadurch realisiert, dass die Umsetzung nach (a) in einer ersten und die Umsetzung nach (b) in einer von der ersten verschiedenen, zweiten Reaktionskolonne durchgeführt werden.
"Reaktionskolonne" bzw. "RD" wird erfindungsgemäß als Abkürzung für
"Reaktivrektifikationskolonne" verwendet.

Bevorzugt enthält die Reaktionskolonne Einbauten. Geeignete Einbauten sind zum Beispiel Böden, strukturierte Packungen oder unstrukturierte Packungen. Wenn die Reaktionskolonne Böden enthält, so sind Glockenböden, Tunnelböden, Thormann-Böden, Kreuzschlitzglockenböden, Ventilböden oder Siebböden geeignet. Wenn die Reaktionskolonne Böden enthält, so werden vorzugsweise solche Böden gewählt, bei denen maximal 5 %, bevorzugt weniger als 1 % der Flüssigkeit durch die jeweiligen Böden durchregnen. Die zur Minimierung des Durchregnens der Flüssigkeit erforderlichen konstruktiven Maßnahmen sind dem Fachmann geläufig. Bei Ventilböden werden zum Beispiel besonders dicht schließende Ventilbauarten gewählt. Durch Verringerung der Zahl der Ventile lässt sich zudem die Dampfgeschwindigkeit in den Bodenöffnungen auf bis zu den doppelten Wert erhöhen, der üblicherweise eingestellt wird. Bei Einsatz von Siebböden ist es besonders günstig, die Durchmesser der Bodenöffnungen zu verringern und die Zahl der Öffnungen beizubehalten oder noch zu vergrößern.

Bei Einsatz von strukturierten oder unstrukturierten Packungen sind strukturierte Packungen im Hinblick auf die gleichmäßige Verteilung der Flüssigkeit bevorzugt. Bei dieser Ausführungsform darf zudem in allen Teilen des Kolonnenquerschnitts, die mehr als 2 % des gesamten Kolonnenquerschnitts entsprechen, das gemittelte Verhältnis von Flüssigkeits- zu Dampfstrom hinsichtlich der Flüssigkeit um nicht mehr als 15 %, bevorzugt um nicht mehr als 3 % überschritten werden. Diese niedrig gehaltene Flüssigkeitsmenge macht es möglich, dass der Kapillareffekt an den Drahtgeweben lokale Spitzenwerte der Flüssigkeitsberieselungsdichte ausschließt.

Bei Kolonnen mit unstrukturierten Packungen, insbesondere mit Füllkörpern, und bei Kolonnen mit strukturierten Packungen kann die gewünschte Charakteristik der Flüssigkeitsverteilung dadurch erzielt werden, dass in dem dem Kolonnenmantel benachbarten Randbereich des Kolonnenquerschnitts, der etwa 2 bis 5 % des gesamten Kolonnenquerschnitts entspricht, die Flüssigkeitsberieselungsdichte gegenüber den übrigen Querschnittsbereichen bis zu 100 %, bevorzugt um 5 bis 15 %, verringert wird. Dies lässt sich zum Beispiel durch die gezielte Verteilung der Abtropfstellen der Flüssigkeitsverteiler oder deren Bohrungen mit einfachen Mitteln erreichen.

In der ersten bevorzugten Ausführungsform werden KOH und NaOH insbesondere jeweils in der oberen Hälfte, bevorzugt dem oberen Drittel, bevorzugter dem oberen Viertel, der jeweiligen Reaktionskolonne zugeführt. Dies ist vorteilhaft, da es eine Verunreinigung des Brüden durch NaOH, KOH oder Alkoholat verhindert.

Von Vorteil in allen bevorzugten Ausführungsformen des erfindungsgemäßen Verfahrens ist es, die NaOH oder KOH-Lösung erwärmt, bevorzugt mit einer Temperatur knapp unterhalb des Siedepunktes, insbesondere 1 bis 10 °C, bevorzugter 1 °C bis 5 °C, am bevorzugtesten 2 bis 3 °C unterhalb des Siedepunktes der jeweiligen Lösung, der Reaktionskolonne zuzuführen. Bei der Verwendung einer wässrigen Natriumhydroxid- oder Kaliumhydroxid-Lösung wird diese vor der Zufuhr in die jeweilige Reaktionskolonne vorzugsweise auf eine Temperatur von 50 bis 70 °C erhitzt, beispielsweise mittels eines Wärmetauschers. Die Zulauftemperatur der Alkalimetallhydroxid-Lösung weist bevorzugt die Temperatur des Bodens an der Zulaufstelle der Alkalimetallhydroxid-Lösung auf.

Der Alkohol ROH kann in allen bevorzugten Ausführungsformen des erfindungsgemäßen Verfahrens sowohl als Dampf als auch flüssig, bevorzugt als Dampf, eingesetzt werden. Bevorzugt wird der Alkohol ROH in den Sumpf, in den Verdampfer (in den bevorzugten Fällen, in denen die jeweilige Reaktionskolonne einen solchen aufweist), auf einen Boden oder auf mehrere Böden, die sich insbesondere 1 bis 10, besonders bevorzugt 1 bis 6 Böden oberhalb des Sumpfes befinden, zugeführt. Insbesondere erfolgt die Zufuhr des Alkohols ROH in dem erfindungsgemäßen Verfahren in den Sumpf oder in den Verdampfer der Reaktionskolonne gasförmig.

"Umsetzung im Gegenstrom" wird erfindungsgemäß insbesondere dadurch gewährleistet, dass der Zugabepunkt mindestens eines Teils des in Schritt (a) eingesetzten Alkohols ROH an der jeweiligen Reaktionskolonne unterhalb des Zugabepunktes von NaOH [in Umsetzung (a)] bzw. KOH [in Umsetzung (b)] liegt. Dies gilt für alle Ausführungsformen der Erfindung. Die Reaktionskolonne, in der das jeweilige Alkalialkoholat NaOR bzw. KOR hergestellt wird, umfasst vorzugsweise mindestens 2, insbesondere 15 bis 40 theoretische Böden zwischen der Zulaufstelle von NaOH bzw. KOH und der Zugabestelle des Alkohols ROH.

Schritt (a) des erfindungsgemäßen Verfahrens umfasst auch den Fall, dass ein Teil des ROH zwar unterhalb des Zugabepunktes der jeweiligen Alkalilauge NaOH bzw. KOH, aber dennoch am oberen Ende oder im Bereich des oberen Endes der jeweiligen Reaktionskolonne dampfförmig zugegeben wird. Dadurch können die Abmessungen im unteren Bereich der jeweiligen Reaktionskolonne verringert werden. Wenn ein Teil des Methanols am oberen Ende oder im Bereich des oberen Endes der Reaktionskolonne dampfförmig zugegeben wird, so wird nur eine Teilmenge von 10 bis 70 Gew.-%, bevorzugt von 30 bis 50 Gew.-% [jeweils bezogen auf die Gesamtmenge des in Schritt (a) bzw. (b) eingesetzten Alkohols] am unteren Ende der Reaktionskolonne eingespeist und die restliche Teilmenge in einem Einzelstrom oder auf mehrere Teilströme verteilt, bevorzugt 1 bis 10 theoretische Böden, besonders bevorzugt 1 bis 3 theoretische Böden unterhalb der jeweiligen Zulaufstelle von NaOH bzw. KOH dampfförmig zugegeben. Dies gilt für alle bevorzugten Ausführungsformen des erfindungsgemäßen Verfahrens.

In der ersten bevorzugten Ausführungsform des erfindungsgemäßen Verfahrens wird am Kopf jeder Reaktionskolonne jeweils ein Brüden erhalten: **B₁,** welcher am Kopf der Reaktionskolonne, in welcher NaOH und ROH im Gegenstrom umgesetzt werden, erhalten wird, umfasst Wasser und ROH. **B₂,** welcher am Kopf der Reaktionskolonne, in welcher KOH und ROH im Gegenstrom umgesetzt werden, erhalten wird, umfasst Wasser und ROH.

In der ersten bevorzugten Ausführungsform des erfindungsgemäßen Verfahrens können beide Reaktionskolonnen mit oder ohne Rücklauf betrieben werden. In einer bevorzugten Ausführungsform weist die erste und/oder die zweite, bevorzugt beide, der voneinander verschiedenen Reaktionskolonnen einen Rücklauf auf. Das heißt, dass **B₁** und/oder **B₂** mindestens teilweise, bevorzugt teilweise, wieder als Rücklauf der Reaktionskolonne zugeführt werden. In den Fällen, in denen ein solcher Rücklauf eingestellt wird, beträgt das Rücklaufverhältnis dabei bevorzugt mindestes 0.05 bis 0.99, bevorzugter 0.1 bis 0.9, noch bevorzugter 0.11 bis 0.34, besonderes bevorzugt 0.14 bis 0.27 und ganz besonders bevorzugt 0.17 bis 0.24. Ein Rücklauf kann dadurch eingestellt werden, dass am Kopf der jeweiligen Kolonne ein Kondensator angebracht wird, in welchem der jeweilige Brüden **B₁** bzw. **B₂** mindestens teilweise kondensiert wird und der jeweiligen RD wieder zugeleitet wird. Unter einem Rücklaufverhältnis wird allgemein und im Sinne dieser Erfindung das Verhältnis des Massenstroms (kg/h) verstanden, der der jeweiligen Kolonne in flüssiger Form zurückgeführt wird (Rücklauf) und des Massenstroms (kg/h), der in flüssiger Form (Destillat) oder gasförmiger Form (Brüden) von der jeweiligen Kolonne abgezogen wird.

In der Ausführungsform, in der an der Reaktionskolonne ein Rücklauf eingestellt wird, kann die in den Schritten (a) bzw. (b) eingesetzte NaOH oder KOH auch mit dem Rücklaufstrom vermischt werden und die resultierende Mischung dann dem jeweiligen Schritt zugeführt werden.

**B₁** und **B₂** werden erfindungsgemäß mindestens teilweise zu **B_{M}** gemischt und aus diesem wird ROH mindestens teilweise durch Destillation von **B_{M}** abgetrennt und so zurückgewonnen. Die mindestens teilweise destillative Abtrennung von ROH aus **B_{M}** wird bevorzugt in einer Rektifikationskolonne durchgeführt, die bevorzugt einen Rücklauf aufweist.

Als Rektifikationskolonne im erfindungsgemäßen Verfahren kann jede beliebige, dem Fachmann bekannte Destillationskolonne eingesetzt werden. Üblicherweise eingesetzte Destillationskolonnen weisen Einbauten auf. Geeignete Einbauten sind zum Beispiel Böden, unstrukturierte Packungen oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten weitere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne möglichst gering bleibt und somit die mechanische Leistung des Verdichters und die Temperatur des zu verdampfenden Methanol/Wasser-Gemischs gering bleibt.

Wenn in der Kolonne strukturierte Packungen oder unstrukturierte Packungen enthalten sind, so können diese geteilt sein oder es kann eine durchgehende Packung vorliegen. Üblicherweise sind jedoch mindestens zwei Packungen vorgesehen, eine Packung oberhalb der Zulaufstelle des ROH/Wasser-Gemischs **B_{M}** und eine Packung unterhalb der Zulaufstelle des ROH/Wasser-Gemischs **B_{M}.** Wenn eine unstrukturierte Packung eingesetzt wird, beispielsweise eine Füllkörperpackung, so liegen die Füllkörper üblicherweise auf einem geeigneten Siebboden oder Gitterboden auf.

Die mindestens teilweise Mischung von **B₁** und **B₂** zu **B_{M}** erfolgt dabei insbesondere dadurch, dass **B₁** und **B₂** aus dem Reaktionsraum der jeweiligen RD geleitet werden und in einer Rektifikationskolonne mindestens teilweise zu **B_{M}** gemischt werden oder vorher mindestens teilweise zu **B_{M}** gemischt werden und **B_{M}** dann der Rektifikationskolonne zugeleitet wird. Noch bevorzugter werden sie vorher mindestens teilweise zu **B_{M}** gemischt und **B_{M}** dann der Rektifikationskolonne zugeleitet.

Bevorzugt wird in der Rektifikationskolonne **B_{M}** in Wasser und Alkohol ROH aufgetrennt, wobei der durch die Trennstufe gewonnene Alkohol ROH bevorzugt der Reaktionskolonne als zurückgeführter Alkohol ROH zugeführt wird.

Der Reaktionskolonne in der ersten bevorzugten Ausführungsform des erfindungsgemäßen Verfahrens kann sowohl mit zurückgeführtem Alkohol ROH aus der Rektifikationskolonne als auch mit Frischalkohol ROH betrieben werden. Bevorzugt wird der Reaktionskolonne sowohl zurückgeführter Alkohol ROH als auch Frischalkohol ROH zugeführt. Wahlweise kann der Frischalkohol der Reaktionskolonne in einer separaten Zulaufstelle oder zusammen mit dem zurückgeführten Alkohol der Reaktionskolonne zugeführt werden.

Der Alkohol ROH, bei dem es sich insbesondere um Frischalkohol ROH, zurückgeführten Alkohol ROH, oder einer Mischung aus beiden, bevorzugt aber um zurückgeführtem Alkohol ROH handelt, wird in der ersten bevorzugten Ausführungsform des erfindungsgemäßen Verfahrens vorzugsweise gasförmig mindestens zwanzig Böden unterhalb der Zulaufstelle der Alkalimetallhydroxid-Lösung der Reaktionskolonne zugeführt. Bevorzugt wird der, bevorzugt zurückgeführte, Alkohol 1 bis 10 Böden, besonders bevorzugt 1 bis 6 Böden oberhalb des Sumpfes oder direkt in den Sumpf der Reaktionskolonne dosiert. Ganz besonders bevorzugt wird der, bevorzugt zurückgeführte, Alkohol ROH unterhalb des untersten Bodens der jeweiligen Reaktionskolonne dosiert.

Die Rektifikationskolonne weist bevorzugt einen Rücklauf, noch bevorzugter einen Kondensator, auf. Das Rücklaufverhältnis beträgt bevorzugter 0.5 bis 2, noch bevorzugter 0.8 bis 1.6. In einer bevorzugten Ausführungsform der ersten bevorzugten Ausführungsform des erfindungsgemäßen Verfahrens wird Frischalkohol ROH in den oberen Teil der Rektifikationskolonne, insbesondere zusammen mit dem Rücklauf der Rektifikationskolonne, zugegeben. Der Alkohol ROH kann entweder im Verstärkungsteil der Rektifikationskolonne oder direkt an deren Kopf zugeführt werden. Die optimale Zulaufstelle ist abhängig vom Wassergehalt des eingesetzten Alkohols ROH und andererseits von dem gewünschten Restwassergehalt im Destillat. Je höher der Wasseranteil im eingesetzten Alkohol und je höher die Reinheitsanforderung im Destillat ist, um so günstiger ist ein Zulauf einige theoretische Böden unterhalb des Kopfes der Rektifikationskolonne. Bevorzugt sind bis zu 20 theoretische Böden unterhalb des Kopfes der Rektifikationskolonne und insbesondere 1 bis 5 theoretische Böden.

Diese Ausführungsform der ersten bevorzugten Ausführungsform des erfindungsgemäßen Verfahrens eignet sich insbesondere, um überschüssiges Wasser aus dem Alkohol ROH zu entfernen, bevor der Alkohol in die Reaktionskolonne rückgeführt wird.

Wenn in der ersten bevorzugten Ausführungsform des erfindungsgemäßen Verfahrens die Rektifikationskolonne mindestens einen Kondensator aufweist, erfolgt die Zugabe des Frischalkohols besonders bevorzugt in den Kondensator oder in den Kondensatbehälter an der Rektifikationskolonne, ganz besonders bevorzugt erfolgt die Zugabe des Frischalkohols dann in den Kondensator an der Rektifikationskolonne.

In der ersten bevorzugten Ausführungsform des erfindungsgemäßen Verfahrens wird der Frischalkohol insbesondere in den Rücklauf der jeweiligen Reaktionskolonne gegeben, dies kann hierbei in den Kondensator als auch in den Kondensatbehälter an der jeweiligen Reaktionskolonne erfolgen.

In einer weiteren bevorzugten Ausführungsform der ersten bevorzugten Ausführungsform des erfindungsgemäßen Verfahrens ist eine der beiden Reaktionskolonnen als Seitenkolonne der anderen ausgestaltet. Dabei weist diejenige der beiden Kolonnen, die nicht als Seitenkolonne ausgestaltet ist, insbesondere ein Verstärkungsteil auf, welcher wiederum bevorzugt bis zu 5 theoretische Böden, noch bevorzugter 1 bis 4 theoretische Böden, am bevorzugtesten 2 bis 3 theoretische Böden umfasst. Der Verstärkungsteil bietet den Vorteil, den Mitriss von Flüssigkeit in den Mischbrüden zu verhindern.

In Abbildung 1 ist eine Ausführungsform <100> gemäß der ersten bevorzugten Ausführungsform des erfindungsgemäßen Verfahrens gezeigt. Es umfasst zwei Reaktionskolonnen <101 > und <102>. Im oberen Fünftel der Reaktionskolonne <101 > wird eine wässrige NaOH-Lösung <1011> zugegeben, im oberen Fünftel der Reaktionskolonne <102> wird eine wässrige KOH-Lösung <1012> zugegeben. Alternativ können auch eine methanolische NaOH-Lösung bzw. eine methanolische KOH-Lösung zugegeben werden. Oberhalb des Sumpfes der Reaktionskolonnen wird Methanol zugegeben (<1021> bei der Reaktionskolonne <101 > und <1022> bei der Reaktionskolonne <102>). Am Sumpf der Reaktionskolonne <101 > wird das Produkt Natriummethanolat <1013> erhalten. Am Sumpf der Reaktionskolonne <102> wird das Produkt Kaliummethanolat <1023> erhalten. Mit dem Verdampfer <1014> bzw. <1024> am Sumpf der jeweiligen Kolonne wird die Konzentration der jeweiligen Alkalimethylatlösung auf den gewünschten Wert eingestellt. Am Kopf der beiden Reaktionskolonnen <101 > und <102> wird jeweils ein Brüden **B₁** <1015> bzw. ein Brüden **B₂** <1025> entnommen. Am Kopf der beiden Reaktionskolonnen <101 > und <102> wird jeweils ein Teil des Brüdenstromes im Kondensator <1016> bzw. <1026> kondensiert und flüssig als Rücklauf auf den Kopf der Reaktionskolonne <101 > bzw. <102> aufgegeben. Der Teil des Brüden **B₁** <1015>, welcher nicht als Rücklauf in die Kolonne <101 > aufgegeben wird, wird demnach über die Leitung <1017> geleitet. Der Teil des Brüden **B₂** <1025>, welcher nicht als Rücklauf in die Kolonne <102> aufgegeben wird, wird demnach über die Leitung <1027> geleitet. Die jeweiligen Teile der beiden Brüden **B₁** <1015> und **B₂** <1025> vermischen sich in der Leitung <1032> zum Mischbrüden **B_{M}** <1031 >. Dieser wird der Rektifikationskolonne <103> zugeleitet. Dort erfolgt die Destillation von Methanol <1039>, welches über Kopf abgeführt wird. Auch am Kopf der Rektifikationskolonne <103> befindet sich ein Kondensator <1036>, über den ein Teil des Methanols aus <1039> kondensiert wird und flüssig am Kopf der Rektifikationskolonne <103> aufgegeben wird. Das übrige Methanol wird dann über den Verdichter <1035> wieder den Reaktionskolonnen zugeführt (<1021> bei der Reaktionskolonne <101 > und <1022> bei der Reaktionskolonne <102>). Es kann dabei auch mit frischem Methanol vermischt werden. Der Verdichter <1035> kann alternativ auch in der Leitung <1032>, in der der Mischbrüden **B_{M}** <1031 > in die Rektifikationskolonne <103> geleitet wird, angebracht sein. Am Sumpf der Kolonne <103> wird Wasser <1033> entnommen. Die Beheizung der Kolonne <103> erfolgt mit Hilfe des Verdampfers <1034>.

In Abbildung 2 ist eine weitere Ausführungsform <200> gemäß der ersten bevorzugten Ausführungsform des erfindungsgemäßen Verfahrens gezeigt. Diese entspricht der in Abbildung 1 gezeigten Ausführungsform und unterscheidet sich darin, dass die Reaktionskolonne für Kaliummethanolat <102> als Seitenkolonne der Reaktionskolonne für Natriummethanolat <101> ausgebildet ist. Im oberen Viertel der Reaktionskolonne <101> wird eine wässrige NaOH-Lösung <1011 > zugegeben, im oberen Fünftel der Seitenkolonne <102> wird eine wässrige KOH-Lösung <1012> zugegeben. Der Brüden **B₂** <1025> vermischt sich deshalb schon in der Kolonne <101> mit dem Brüden **B₁** <1015> (durch geschwungene Pfeile symbolisiert). Dadurch wird schließlich ein Mischbrüden **B_{M}** <1031 > erhalten, der mindestens teilweise am Kopf der Kolonne <101> über die Leitung <1032> entnommen wird. Die Kolonne <101> kann optional einen Verstärkungsteil <1040> mit bevorzugt bis zu 5 theoretischen Stufen aufweisen, um einen Mitriss von Flüssigkeit in den Mischbrüden **B_{M}** <1031 > zu vermeiden. Des Weiteren kann ein Rücklauf über den Kondensator <1016> in die Kolonne <101 > geleitet werden. Über Leitung <1018> kann ein Flüssigkeitsstrom aus der Kolonne <101 > ganz oder teilweise in die Kolonne <102> geleitet werden. Die Bedeutung der Zeichen <103>, <1013>, <1014>, <1021>, <1022>, <1023>, <1024>, <1033>, <1034>, <1035>, <1036> und <1039> in Abbildung 2 sind wie für Abbildung 1 angegeben.

### 2. Zweite bevorzugte Ausführungsform: (a) und (b) in einer Reaktionskolonne

In einer zweiten bevorzugten Ausführungsform des erfindungsgemäßen Verfahrens wird die räumliche Trennung der beiden Umsetzungen (a) und (b) dadurch realisiert, dass die Umsetzung nach (a) und die Umsetzung nach (b) in derselben Reaktionskolonne, in welcher sie durch mindestens eine Trennwand voneinander getrennt sind, durchgeführt werden. Solche Reaktionskolonnen werden im Folgenden als Trennwand-Reaktionskolonnen bezeichnet. "Trennwand-Reaktionskolonnen" werden im Folgenden als "TRD" abgekürzt. Die mindestens eine in der zweiten bevorzugten Ausführungsform des erfindungsgemäßen Verfahrens eingesetzte TRD weist bevorzugt einen Verstärkungsteil oberhalb der Trennwand auf.

TRDs werden im Prinzip dadurch aus den herkömmlichen Reaktionskolonnen erhalten, dass in ihnen eine Trennwand eingezogen wird, wodurch zwei separate Reaktionsräume entstehen. Solche Trennwandkolonnen sind dem Fachmann bekannt und beispielsweise beschrieben in US 2,295,256, EP 0 122 367 A2, EP 0 126 288 A2, WO 2010/097318 A1 sowie von I. Dejanović, Lj. Matijašević, Ž. Olujić, *Chemical Engineering and Processing* **2010,** *49*, 559-580. In den für das erfindungsgemäße Verfahren in Frage kommenden Trennwandkolonnen sind die Trennwände bevorzugt bis zum Boden durchgezogen und durchspannen insbesondere bevorzugt mindestens ein Viertel, bevorzugter mindestens ein Drittel, noch bevorzugter mindestens die Hälfte, noch bevorzugter mindesten zwei Drittel, noch mehr bevorzugter mindestens drei Viertel der Kolonne der Länge nach. Sie teilen die Kolonne in mindestens zwei Reaktionsräume, in denen räumlich getrennte Reaktionen stattfinden können. Die durch die mindestens eine Trennwand geschaffenen Reaktionsräume können gleich oder unterschiedlich groß sein.

Die Reaktionskolonne, welche in der zweiten bevorzugten Ausführungsform des erfindungsgemäßen Verfahrens eingesetzt wird, umfasst mindestens eine Trennwand, bevorzugt eine Trennwand oder zwei Trennwände. Insbesondere weisen TRDs auch einen Rücklauf auf.

In der zweiten bevorzugten Ausführungsform des erfindungsgemäßen Verfahrens werden KOH und NaOH insbesondere jeweils in der oberen Hälfte, bevorzugt dem oberen Drittel, bevorzugter dem oberen Viertel, des von der Trennwand durchspannten Bereichs der Reaktionskolonne zugeführt. Dies gewährleistet, dass das jeweilige Alkalimetallhydroxid nur dem Reaktionsraum zugegeben wird, in dem es reagieren soll und so Kreuzkontamination vermieden wird. Gleichzeitig sorgt die Zugabe nahe am oberen Rand (aber unterhalb des oberen Randes) für eine möglichst lange Reaktionszeit des jeweiligen Alkalimetallhydroxids mit ROH.

TRDs haben den Vorteil, dass sie platzsparender sind und sich die beiden Brüden **B₁** und **B₂** im Raum oberhalb der Trennwand mindestens teilweise zu **B_{M}** mischen.

In der zweiten bevorzugten Ausführungsform des erfindungsgemäßen Verfahrens wird insbesondere die mindestens teilweise destillative Abtrennung von ROH aus **B_{M}** dann in einer von der TRD verschiedenen Rektifikationskolonne durchgeführt. Diese Rektifikationskolonne weist bevorzugt einen Rücklauf auf, wie er für die Rektifikationskolonne nach der ersten bevorzugten Ausführungsform angegeben ist.

Alternativ weist die TRD in dieser zweiten bevorzugten Ausführungsform des erfindungsgemäßen Verfahrens insbesondere einen Verstärkungsteil auf, wobei die Rektifikationskolonne als Seitenkolonne der TRD ausgestaltet ist

In einer weiteren alternativen bevorzugten Ausführungsform gemäß der zweiten bevorzugten Ausführungsform des erfindungsgemäßen Verfahrens werden die Umsetzung nach (a), die Umsetzung nach (b) und die destillative Abtrennung von ROH aus **B_{M}** in derselben Reaktionskolonne, in welcher sie durch zwei Trennwände voneinander getrennt sind, durchgeführt.

Durch die beiden Trennwände entstehen drei voneinander getrennte Räume, von denen einer für den Schritt (a), einer für den Schritt (b) und einer für die destillative Abtrennung von ROH aus **B_{M}** genutzt wird.

Dadurch kann **B_{M}** mindestens teilweise in derselben TRD durch Schaffung eines weiteren Raumes mittels einer zweiten Trennwand in Wasser und ROH destillativ getrennt und ROH so zurückgewonnen werden. Die Umsetzung nach (a), die Umsetzung nach (b) und die mindestens teilweise destillative Abtrennung von ROH aus **B_{M}** werden so in derselben Reaktionskolonne, in welcher sie durch zwei Trennwände (die drei abgetrennte Bereiche schaffen) voneinander getrennt sind, durchgeführt.

In Abbildung 3 ist eine Ausführungsform <300> gemäß der zweiten bevorzugten Ausführungsform des erfindungsgemäßen Verfahrens gezeigt. Es umfasst eine Reaktionskolonne <105>, welche eine bis zum Boden der Kolonne durchgezogene, senkrechte Trennwand <1053> umfasst, so dass sich in der Kolonne zwei Reaktionsräume <1051 > und <1052> ausbilden.

In den Reaktionsraum <1051 > wird eine wässrige NaOH-Lösung <1011 > zugegeben. In den Reaktionsraum <1052> wird eine wässrige KOH-Lösung <1012> zugegeben. Alternativ können auch jeweils eine methanolische NaOH-Lösung bzw. eine methanolische KOH-Lösung zugegeben werden. Die Zugabepunkte befinden sich unterhalb der Höhe, welche das obere Ende der Trennwand <1053> definiert, da so vermieden wird, dass das jeweilige Alkalimetallhydroxid in den verkehrten Reaktionsraum läuft. Oberhalb des Sumpfes der Reaktionskolonnen wird Methanol zugegeben (<1021> beim Reaktionsraum <1051 > und <1022> beim Reaktionsraum <1052>). Am Sumpf der Reaktionskolonne <105> werden das Produkt Natriummethanolat <1013> und das Produkt Kaliummethanolat <1023> erhalten. Mit den Verdampfern <1014> und <1024> am Sumpf der Kolonne werden die Konzentrationen der beiden Alkoholatlösungen auf den gewünschten Wert eingestellt. In der Reaktionskolonne <105> wird aus der Mischung der beiden Brüden der Mischbrüden **B_{M}** <1031 > erhalten und mindestens teilweise über die Leitung <1032> am Kopf der Kolonne <101 > entnommen. Am Kopf der Reaktionskolonne <105> befindet sich ein Kondensator <1016>, über welchen ein Rücklauf in die Kolonne <105> geleitet werden kann. Die Brüdenmischung **B_{M}** <1031 > wird der Rektifikationskolonne <103> mindestens teilweise über die Leitung <1032> zugeleitet. Dort erfolgt die destillative Abtrennung von Methanol <1039>, welches über Kopf abgeführt wird. Auch am Kopf der Rektifikationskolonne <103> befindet sich ein Kondensator <1036>, über den ein Teil des Methanols <1039> kondensiert wird und am Kopf der Kolonne <103> aufgegeben wird. Am Kondensator <1036> kann frisches Methanol <1037> zugeleitet werden. Frisches Methanol <1037> kann auch in den Ausführungsformen des erfindungsgemäßen Verfahrens, welches in den Abbildungen 1 und 2 gezeigt sind, zugegeben werden. Das übrige Methanol wird dann wieder über den Verdichter <1035> der Reaktionskolonne <105> zugeführt. Es kann dabei auch mit frischem Methanol <1037> vermischt werden. Der Verdichter <1035> kann alternativ auch in der Leitung <1032>, in der mindestens ein Teil der Brüdenmischung **B_{M}** <1031 > in die Rektifikationskolonne <103> geleitet wird, angebracht sein, noch bevorzugter in dem Teil der Leitung <1032>, der von mindestens einem Teil der Brüdenmischung **B_{M}** <1031> nach Passieren der Abzweigung zum Kondensator <1016> durchflossen wird.

Am Sumpf der Kolonne <103> wird Wasser <1033> entnommen. Die Beheizung der Kolonne <103> erfolgt mit Hilfe des Verdampfers <1034>.

In Abbildung 4 ist eine weitere Ausführungsform <400> gemäß der zweiten bevorzugten Ausführungsform des erfindungsgemäßen Verfahrens gezeigt. Diese entspricht der in Abbildung 3 gezeigten Ausführungsform und unterscheidet sich darin, dass der Abtriebsteil der Destillationskolonne <103> als Seitenkolonne der Trennwandkolonne <105> ausgeführt ist und der Verstärkungsteil <1040> in die Kolonne <105> integriert ist. Der Brüden **B₂** <1025> vermischt sich deshalb schon in der Kolonne <105> mit dem Brüden **B₁** <1015> und dem Brüden <10311 > aus Kolonne <103>. Die Rückgewinnung des Methanols aus den Brüden der Reaktionsabschnitte <1051 > und <1052> erfolgt durch Destillation im Verstärkungsteil von <105> und die Abtrennung von Wasser erfolgt in der Seitenkolonne <103>. Die Kolonnen <105> und <103> sind über den Flüssigkeitsstrom <10312> und den Brüdenstrom <10311 > verbunden. Das erhaltene Methanol wird dann über Leitung <1032> teilweise der Kolonne <105> entnommen und via <1021 > und <1022> in den Reaktionsabschnitten wiederverwendet. Der andere Teil wird für den Rücklauf, der über den Kondensator <1036> in die Kolonne <105> rückgeführt wird, verwendet.

Die Bedeutung der Zeichen <1011>, <1012>, <1013>, <1014>, <1023>, <1024>, <1031>, <1033>, <1034>, <1035>, <1037>, <1051>, <1052> und <1053> in Abbildung 4 sind wie für Abbildung 3 angegeben.

In Abbildung 5 ist eine weitere Ausführungsform <500> gemäß der zweiten bevorzugten Ausführungsform des erfindungsgemäßen Verfahrens gezeigt. Diese entspricht den in Abbildungen 3 und 4 gezeigten Ausführungsformen und unterscheidet sich durch eine Reaktionskolonne <601> mit zwei Trennwänden <1053> und <1056>. Durch die Ausbildung zweier Trennwände <1053> und <1056> wird der untere Teil der Kolonne in drei getrennte Räume unterteilt: die zwei Reaktionsräume <1051>, <1052> und der Abtriebsteil <1055>. In den Reaktionsräumen <1051 > und <1052> läuft jeweils die Umsetzung der wässrigen NaOH-Lösung <1011 > bzw. der wässrigen KOH-Lösung <1012> mit Methanol (<1021> beim Reaktionsraum <1051> und <1022> beim Reaktionsraum <1052>) zu Natriummethylat bzw. Kaliummethylat ab, während im Abtriebsteil <1055> die Trennung von Methanol und Wasser abläuft.

Die Bedeutung der Zeichen <1011>, <1012>, <1013>, <1014>, <1021>, <1022>, <1023>, <1024>, <1033>, <1034>, <1035>, <1036>, <1037> und <1040> in Abbildung 5 sind wie für Abbildung 3 angegeben.

### 3. Dritte bevorzugte Ausführungsform: Destillation und einer der Schritte (a), (b) in einer Reaktionskolonne, der übrige Schritt in der Seitenkolonne

In einer dritten bevorzugten Ausführungsform des erfindungsgemäßen Verfahrens wird dieses in einer TRD und einer Seitenkolonne durchgeführt, wobei die TRD bevorzugt einen Verstärkungsteil umfasst. Die TRD umfasst eine Trennwand, wodurch zwei durch die Trennwand abgegrenzte Bereiche in der TRD entstehen.

In dieser Ausführungsform erfolgt einer der beiden Schritte (a) oder (b) des erfindungsgemäßen Verfahrens in einem der durch die Trennwand abgegrenzten Bereiche der TRD, und der andere der beiden Schritte in der Seitenkolonne. Die mindestens teilweise destillative Abtrennung von ROH aus **B_{M}** erfolgt in dem durch die Trennwand abgegrenzten Bereich der TRD, in dem keiner der Schritte (a), (b) des erfindungsgemäßen Verfahrens durchgeführt wird, und in den bevorzugten Fällen, in denen die TRD dieses umfasst, im Verstärkungsteil.

Diese Ausführungsform ermöglicht beim Einsatz einer TRD eine bessere räumliche Trennung der Schritte (a) und (b), da einer davon in der TRD und der andere in der Seitenkolonne durchgeführt wird.

### Beispiele

### Beispiel 1 (nicht erfindungsgemäß): Verwendung nur einer Reaktionskolonne, ohne Waschen

Die Reaktionskolonne wird zunächst kontinuierlich mit einem Strom wässriger NaOH aus einem ersten Alkalitank (Tank 1) und Methanol im Gegenstrom betrieben. Das hierbei produzierte Natriummethanolat (gelöst in Methanol) verlässt die Reaktionskolonne am Sumpf und weist nur sehr geringe Mengen an NaOH und Wasser auf (ca. 1000 ppm). Ein Methanol-Wasser-Gemisch verlässt die Reaktionskolonne dampfförmig am Kopf und wird kontinuierlich der nachfolgenden Rektifikationskolonne zugeführt. Hier wird das Methanol-Wasser-Gemisch aufgetrennt und ein nahezu wasserfreier Methanolstrom am Kopf sowie ein nahezu methanolfreier Wasserstrom am Sumpf der Kolonne erhalten. Das rückgewonnene Methanol wird dampfförmig zur Reaktionskolonne zurückgeführt und dort oberhalb des Sumpfes eingeleitet.

Zur anschließenden Herstellung von Kaliummethanolat wird die Anlage abgefahren und der Sumpf der Reaktionskolonne entleert, um die darin enthaltene Natriummethanolat-Lösung zu entnehmen. Dieser Vorgang dauert einige Stunden, in denen keine Produktion erfolgen kann. Ein weiteres Spülen der Reaktionskolonne erfolgt aus Zeitgründen nicht. Die Anlage wird anschließend mit einem Zulauf wässriger KOH aus einem zweiten Alkalitank (Tank 2) zur Reaktionskolonne wieder angefahren. Die am Sumpf der Reaktionskolonne entnommene Kaliummethanolat-Lösung enthält neben dem Rest aus nicht reagierter KOH und Wasser nun auch die Reste an NaOH und Natriummethanolat aus den Einbauten der Kolonne. Hierdurch weist die Kaliummethanolat-Lösung zunächst eine sehr hohe Verunreinigung auf und muss daher verworfen werden. Durch Verdünnung erreicht die Verunreinigung erst nach einiger Zeit akzeptable Werte (< 5000 ppm). Ab diesem Zeitpunkt wird zwar verkaufsfähiges Produkt produziert, jedoch mit deutlich schlechterer Qualität als vor dem Produktwechsel (>> 1000 ppm). Ein anschließender, erneuter Wechsel zur Produktion von Natriummethanolat ist mit den gleichen Schritten verbunden.

### Beispiel 2 (nicht erfindungsgemäß): Verwendung nur einer Reaktionskolonne, mit Waschen

Die Reaktionskolonne wird zunächst kontinuierlich mit einem Strom wässriger NaOH aus einem ersten Alkalitank (Tank 1) und Methanol im Gegenstrom betrieben. Das hierbei produzierte Natriummethanolat (gelöst in Methanol) verlässt die Reaktionskolonne am Sumpf und weist nur sehr geringe Mengen an NaOH und Wasser auf (ca. 1000 ppm). Ein Methanol-Wasser-Gemisch verlässt die Reaktionskolonne dampfförmig am Kopf und wird kontinuierlich der nachfolgenden Rektifikationskolonne zugeführt. Hier wird das Methanol-Wasser-Gemisch aufgetrennt und ein nahezu wasserfreier Methanolstrom am Kopf sowie ein nahezu methanolfreier Wasserstrom am Sumpf der Kolonne erhalten. Das rückgewonnene Methanol wird dampfförmig zur Reaktionskolonne zurückgeführt und dort oberhalb des Sumpfes eingeleitet.

Zur anschließenden Herstellung von Kaliummethanolat wird die Anlage abgefahren und der Sumpf der Reaktionskolonne entleert, um die darin enthaltene Natriummethanolat-Lösung zu entnehmen. Dieser Vorgang dauert einige Stunden, in denen keine Produktion erfolgen kann. Im Anschluss wird die Anlage gründlich gereinigt, um die Verunreinigung des Kaliummethanolats durch Reste in der Reaktionskolonne zu vermeiden. Hierzu wird die Reaktionskolonne mehrmals hintereinander mit Wasserdampf gespült und entleert. Dieser Vorgang dauert ebenfalls mehrere Stunden, in denen keine Produktion erfolgen kann. Hierbei werden außerdem große Mengen an Wasserdampf verbraucht. Die Anlage wird anschließend mit einem Zulauf wässriger KOH aus einem zweiten Alkalitank (Tank 2) zur Reaktionskolonne wieder angefahren. Die am Sumpf der Reaktionskolonne entnommene Kaliummethanolat-Lösung weist nur sehr geringe Mengen an KOH und Wasser auf sowie sehr geringe Reste an NaOH und Natriummethanolat auf. Diese Lösung ist aufgrund des Spülens direkt verkaufsfähig, weist jedoch immer noch leichte Verunreinigungen an NaOH und Natriummethanolat auf.

### Beispiel 3 (erfindungsgemäß), entspricht Abbildung 1

In zwei gleichzeitig und kontinuierlich betriebenen Reaktionskolonnen erfolgt die Produktion von Natrium- und Kaliummethanolat. Die erste Reaktionskolonne <101 > wird kontinuierlich mit einem Strom wässriger NaOH <1011> aus einem ersten Alkalitank (Tank 1) und Methanol <1021> im Gegenstrom betrieben. Das hierbei produzierte Natriummethanolat (gelöst in Methanol) verlässt die Reaktionskolonne am Sumpf <1013> und weist nur sehr geringe Mengen an NaOH und Wasser auf (ca. 1000 ppm). Ein Methanol-Wasser-Gemisch <1015> verlässt die Reaktionskolonne dampfförmig am Kopf. Die zweite Reaktionskolonne <102> wird kontinuierlich mit einem Strom wässriger KOH <1012> aus einem zweiten Alkalitank (Tank 2) und Methanol <1022> im Gegenstrom betrieben. Das hierbei produzierte Kaliummethanolat (gelöst in Methanol) verlässt die Reaktionskolonne am Sumpf <1023> und weist nur sehr geringe Mengen an KOH und Wasser auf (ca. 1000 ppm). Ein Methanol-Wasser-Gemisch <1025> verlässt die Reaktionskolonne dampfförmig am Kopf. Beide Methanol-Wasser-Gemische am Kopf der Reaktionskolonnen werden vermischt und gemeinsam kontinuierlich der nachfolgenden Rektifikationskolonne <103> als Mischung <1031 > zugeführt. Hier wird das Methanol-Wasser-Gemisch <1031 > aufgetrennt und ein nahezu wasserfreier Methanolstrom <1039> am Kopf sowie ein nahezu methanolfreier Wasserstrom <1033> am Sumpf der Kolonne erhalten. Das rückgewonnene Methanol wird dampfförmig zu beiden Reaktionskolonnen zurückgeführt und dort jeweils oberhalb des Sumpfes eingeleitet.

### Beispiel 4 (erfindungsgemäß): entspricht Abbildung 3

Die Reaktionskolonne <105> weist eine mittig platzierte, nach unten durchgezogene Trennwand <1053> auf. Hierdurch werden zwei räumlich getrennte Reaktionsräume <1051 > und <1052> gebildet, in denen zwei Reaktionen simultan ablaufen können. Oberhalb der Trennwand befinden sich lediglich drei Ventilböden. Auf die erste Seite der Trennwand wird kontinuierlich ein Strom wässriger NaOH <1011 > aus einem ersten Alkalitank (Tank 1) und Methanol <1021 > im Gegenstrom zugeführt. Das hierbei produzierte Natriummethanolat (gelöst in Methanol) verlässt die Reaktionskolonne am Sumpf <1013> und weist nur sehr geringe Mengen an NaOH und Wasser auf (ca. 1000 ppm). Auf die zweite Seite der Trennwand wird kontinuierlich ein Strom wässriger KOH <1012> aus einem zweiten Alkalitank (Tank 2) und Methanol <1022> im Gegenstrom zugeführt. Das hierbei produzierte Kaliummethanolat (gelöst in Methanol) verlässt die Reaktionskolonne am Sumpf <1023> und weist nur sehr geringe Mengen an KOH und Wasser auf (ca. 1000 ppm). Jeweils ein dampfförmiges Methanol-Wasser-Gemisch verlässt am oberen Ende der Trennwand beide Seiten der Trennwand. Diese vermischen sich oberhalb der Trennwand. Das resultierende Methanol-Wasser-Gemisch <1031> verlässt die Reaktionskolonne dampfförmig am Kopf und wir kontinuierlich der nachfolgenden Rektifikationskolonne <103> zugeführt. Hier wird das Methanol-Wasser-Gemisch aufgetrennt und ein nahezu wasserfreier Methanolstrom <1039> am Kopf sowie ein nahezu methanolfreier Wasserstrom <1033> am Sumpf der Kolonne erhalten. Das rückgewonnene Methanol wird dampfförmig zu der Reaktionskolonne rückgeführt, aufgeteilt und auf beiden Seiten der Trennwand oberhalb der beiden Sümpfe eingeleitet.

### Ergebnis:

Die Verwendung nur einer Reaktionskolonne wie in Beispiel 1 und 2 bringt Nachteile mit sich, nämlich eine Kreuzkontamination von Natriummethylat in Kaliummethylat und umgekehrt, welche nur durch aufwendiges Reinigen vermieden werden konnte. Demgegenüber erlaubt das erfindungsgemäße Verfahren, wie es in den Beispielen 3 und 4 gezeigt ist, eine reine Darstellung der jeweiligen Methylate ohne Produktionsunterbrechung mit hoher Reinheit.

## Patentansprüche

1. Verfahren zur Herstellung von Natriumalkoholat der Formel RONa und Kaliumalkoholat der Formel ROK, wobei
(a) ROH mit NaOH im Gegenstrom umgesetzt wird, wodurch RONa und ein Brüden **B₁** umfassend Wasser und ROH erhalten werden,
(b) ROH mit KOH im Gegenstrom umgesetzt wird, wodurch ROK und ein Brüden **B₂** umfassend Wasser und ROH erhalten werden,
wobei Rein C₁-C₆-Kohlenwasserstoffrest ist,
**dadurch gekennzeichnet,**
**dass** die Umsetzungen nach (a) und (b) gleichzeitig und räumlich getrennt ablaufen,
und **dass** die Brüden **B₁** und **B₂** mindestens teilweise zum gemischten Brüden **B_{M}** umfassend ROH und Wasser gemischt werden,
und **dass** ROH mindestens teilweise durch Destillation aus **B_{M}** abgetrennt wird.

2. Verfahren nach Anspruch 1, **dadurch gekennzeichnet, dass** der aus **B_{M}** abgetrennte Alkohol ROH wieder in Umsetzung (a) und/oder Umsetzung (b) eingesetzt wird.

3. Verfahren nach Anspruch 1 oder 2, **dadurch gekennzeichnet, dass** ROH aus der Gruppe bestehend aus Methanol, Ethanol, *n*-Propanol, *iso*-Propanol, *n*-Butanol, *sec*-Butanol, *iso*-Butanol, *tert*-Butanol, Isomeren des Pentanols ausgewählt ist.

4. Verfahren nach einem der Ansprüche 1 bis 3, **dadurch gekennzeichnet, dass** NaOH in der Umsetzung nach (a) und/oder KOH in der Umsetzung nach (b) in mindestens einem Lösungsmittel ausgewählt aus Wasser, ROH zugegeben wird.

5. Verfahren nach einem der Ansprüche 1 bis 4 **dadurch gekennzeichnet, dass** die Umsetzung nach (a) in einer ersten Reaktionskolonne und die Umsetzung nach (b) in einer von der ersten verschiedenen, zweiten Reaktionskolonne durchgeführt werden.

6. Verfahren nach Anspruch 5, **dadurch gekennzeichnet, dass** KOH und NaOH jeweils in der oberen Hälfte der jeweiligen Reaktionskolonne zugeführt werden.

7. Verfahren nach Anspruch 5 oder 6, **dadurch gekennzeichnet, dass** die erste und/oder die zweite Reaktionskolonne einen Rücklauf aufweist.

8. Verfahren nach einem der Ansprüche 5 bis 7, **dadurch gekennzeichnet, dass** die mindestens teilweise destillative Abtrennung von ROH aus **B_{M}** in einer Rektifikationskolonne durchgeführt wird.

9. Verfahren nach einem der Ansprüche 5 bis 8, **dadurch gekennzeichnet, dass** eine der beiden Reaktionskolonnen als Seitenkolonne der anderen ausgestaltet ist.

10. Verfahren nach einem der Ansprüche 1 bis 4, **dadurch gekennzeichnet, dass** die Umsetzung nach (a) und die Umsetzung nach (b) in derselben Reaktionskolonne, in welcher sie durch mindestens eine Trennwand voneinander getrennt sind, durchgeführt werden.

11. Verfahren nach Anspruch 10, **dadurch gekennzeichnet, dass** KOH und NaOH jeweils in der oberen Hälfte des von der Trennwand durchspannten Bereichs der Reaktionskolonne zugeführt werden.

12. Verfahren nach Anspruch 10 oder 11, **dadurch gekennzeichnet, dass** die mindestens teilweise destillative Abtrennung von ROH aus **B_{M}** in einer von der Reaktionskolonne verschiedenen Rektifikationskolonne durchgeführt wird.

13. Verfahren nach Anspruch 12, **dadurch gekennzeichnet, dass** die Reaktionskolonne einen Verstärkungsteil aufweist und die Rektifikationskolonne als Seitenkolonne der Reaktionskolonne ausgestaltet ist.

14. Verfahren nach Anspruch 10 oder 11, **dadurch gekennzeichnet, dass** die Umsetzung nach (a), die Umsetzung nach (b) und die destillative Abtrennung von ROH aus **B_{M}** in derselben Reaktionskolonne, in welcher sie durch zwei Trennwände voneinander getrennt sind, durchgeführt werden.

15. Verfahren nach einem der Ansprüche 1 bis 4, **dadurch gekennzeichnet, dass** dieses in einer Reaktionskolonne, welche eine Trennwand umfasst, und einer Seitenkolonne der Reaktionskolonne durchgeführt wird, wobei einer der beiden Schritte (a) und (b) in einem der durch die Trennwand abgegrenzten Bereiche der Reaktionskolonne durchgeführt wird, und der andere der beiden Schritte (a) und (b) in der Seitenkolonne durchgeführt wird, und die mindestens teilweise destillative Abtrennung von ROH aus **B_{M}** in dem durch die Trennwand abgegrenzten Bereich der Reaktionskolonne, in dem keiner der Schritte (a), (b) durchgeführt wird, erfolgt.

16. Verfahren nach einem der Ansprüche 1 bis 15, welches kontinuierlich durchgeführt wird.

## Claims

1. Process for producing sodium alkoxide of the formula RONa and potassium alkoxide of the formula ROK, wherein
(a) ROH is reacted with NaOH in countercurrent, whereby RONa and a vapour **B₁** comprising water and ROH are obtained,
(b) ROH is reacted with KOH in countercurrent, whereby ROK and a vapour **B₂** comprising water and ROH are obtained, wherein R is a C₁-C₆-hydrocarbon radical,
**characterized in**
**that** the reactions according to (a) and (b) proceed simultaneously and spatially separated,
and **that** the vapours **B₁** and **B₂** are at least partially mixed to give the mixed vapour **B_{M}** comprising ROH and water,
and **that** ROH is at least partially separated off from **B_{M}** by distillation.

2. Process according to Claim 1, **characterized in that** the alcohol ROH separated off from **B_{M}** is used again in reaction (a) and/or reaction (b).

3. Process according to Claim 1 or 2, **characterized in that** ROH is selected from the group consisting of methanol, ethanol, *n*-propanol, isopropanol, *n*-butanol, sec-butanol, isobutanol, *tert*-butanol, isomers of pentanol.

4. Process according to any of Claims 1 to 3, **characterized in that** NaOH in the reaction according to (a) and/or KOH in the reaction according to (b) is added in at least one solvent selected from water, ROH.

5. Process according to any of Claims 1 to 4, **characterized in that** the reaction according to (a) is carried out in a first reaction column and the reaction according to (b) is carried out in a second reaction column different from the first.

6. Process according to Claim 5, **characterized in that** KOH and NaOH are each fed to the upper half of the respective reaction column.

7. Process according to Claim 5 or 6, **characterized in that** the first and/or the second reaction column has a return flow.

8. Process according to any of Claims 5 to 7, **characterized in that** the at least partial distillative separation of ROH from **B_{M}** is carried out in a rectification column.

9. Process according to any of Claims 5 to 8, **characterized in that** one of the two reaction columns is configured as side column to the other.

10. Process according to any of Claims 1 to 4, **characterized in that** the reaction according to (a) and the reaction according to (b) are carried out in the same reaction column in which they are separated from each other by at least one dividing wall.

11. Process according to Claim 10, **characterized in that** KOH and NaOH are each fed to the upper half of the region of the reaction column spanned by the dividing wall.

12. Process according to Claim 10 or 11, **characterized in that** the at least partial distillative separation of ROH from **B_{M}** is carried out in a rectification column different from the reaction column.

13. Process according to Claim 12, **characterized in that** the reaction column has a rectifying section and the rectification column is configured as side column to the reaction column.

14. Process according to Claim 10 or 11, **characterized in that** the reaction according to (a), the reaction according to (b) and the distillative separation of ROH from **B_{M}** are carried out in the same reaction column in which they are separated from one other by two dividing walls.

15. Process according to any of Claims 1 to 4, **characterized in that** said process is carried out in a reaction column comprising a dividing wall, and a side column to the reaction column, wherein one of the two steps (a) and (b) is carried out in one of the regions of the reaction column segregated by the dividing wall, and the other of the two steps (a) and (b) is carried out in the side column, and the at least partial distillative separation of ROH from **B_{M}** takes place in the region of the reaction column segregated by the dividing wall in which neither of the steps (a), (b) is carried out.

16. Process according to any of Claims 1 to 15, wherein said process is carried out continuously.

## Revendications

1. Procédé pour la production d'alcoolate de sodium de formule RONa et d'alcoolate de potassium de formule ROK, dans lequel
(a) ROH est mis en réaction avec NaOH à contre-courant, ce par quoi on obtient RONa et une vapeur **B₁** comprenant de l'eau et ROH,
(b) ROH est mis en réaction avec KOH à contre-courant, ce par quoi on obtient ROK et une vapeur **B₂** comprenant de l'eau et ROH,
R étant un radical hydrocarboné en C₁-C₆,
**caractérisé en ce que**
les réactions selon (a) et (b) se déroulent simultanément et séparées spatialement,
et **en ce que** les vapeurs **B₁** et **B₂** sont au moins partiellement mélangées en la vapeur mixte **B_{M}** comprenant ROH et de l'eau,
et **en ce que** ROH est au moins partiellement séparé par distillation à partir de **B_{M}.**

2. Procédé selon la revendication 1, **caractérisé en ce que** l'alcool ROH séparé à partir de **B_{M}** est réutilisé dans la réaction (a) et/ou la réaction (b).

3. Procédé selon la revendication 1 ou 2, **caractérisé en ce que** ROH est choisi dans le groupe constitué par le méthanol, l'éthanol, le *n*-propanol, l'*iso*-propanol, le *n*-butanol, le *sec*-butanol, l'*iso*-butanol, le *tert-*butanol, les isomères du pentanol.

4. Procédé selon l'une quelconque des revendications 1 à 3, **caractérisé en ce que** NaOH dans la réaction selon (a) et/ou KOH dans la réaction selon (b) est/sont ajouté(s) dans au moins un solvant choisi parmi l'eau, ROH.

5. Procédé selon l'une quelconque des revendications 1 à 4, **caractérisé en ce que** la réaction selon (a) est effectuée dans une première colonne de réaction et la réaction selon (b) est effectuée dans une deuxième colonne de réaction différente de la première.

6. Procédé selon la revendication 5, **caractérisé en ce que** KOH et NaOH sont introduits chacun dans la moitié supérieure de la colonne de réaction respective.

7. Procédé selon la revendication 5 ou 6, **caractérisé en ce que** la première et/ou la deuxième colonne de réaction comporte(nt) une recirculation.

8. Procédé selon l'une quelconque des revendications 5 à 7, **caractérisé en ce que** ladite au moins partielle séparation de ROH par distillation à partir de **B_{M}** est effectuée dans une colonne de rectification.

9. Procédé selon l'une quelconque des revendications 5 à 8, **caractérisé en ce que** l'une des deux colonnes de réaction est configurée en tant que colonne latérale à l'autre.

10. Procédé selon l'une quelconque des revendications 1 à 4, **caractérisé en ce que** la réaction selon (a) et la réaction selon (b) sont effectuées dans la même colonne de réaction, dans laquelle elles sont séparées l'une de l'autre par au moins une paroi de séparation.

11. Procédé selon la revendication 10, **caractérisé en ce que** KOH et NaOH sont introduits chacun dans la moitié supérieure de la zone traversée par la paroi de séparation de la colonne de réaction.

12. Procédé selon la revendication 10 ou 11, **caractérisé en ce que** ladite au moins partielle séparation de ROH par distillation à partir de **B_{M}** est effectuée dans une colonne de rectification différente de la colonne de réaction.

13. Procédé selon la revendication 12, **caractérisé en ce que** la colonne de réaction comporte un élément de renfort et la colonne de rectification est configurée en tant que colonne latérale à la colonne de réaction.

14. Procédé selon la revendication 10 ou 11, **caractérisé en ce que** la réaction selon (a), la réaction selon (b) et la séparation de ROH par distillation à partir de **B_{M}** sont effectuées dans la même colonne de réaction, dans laquelle elles sont séparées les unes des autres par deux parois de séparation.

15. Procédé selon l'une quelconque des revendications 1 à 4, **caractérisé en ce que** celui-ci est effectué dans une colonne de réaction, qui comprend une paroi de séparation, et une colonne latérale à la colonne de réaction, l'une des deux étapes (a) et (b) étant effectuée dans l'une des zones délimitées par la paroi de séparation de la colonne de réaction, et l'autre des deux étapes (a) et (b) étant effectuée dans la colonne latérale, et ladite au moins partielle séparation de ROH par distillation à partir de **B_{M}** étant effectuée dans la zone délimitée par la paroi de séparation de la colonne de réaction, dans laquelle n'est effectuée aucune des étapes (a), (b).

16. Procédé selon l'une quelconque des revendications 1 à 15, qui est effectué en continu.
